# EUROPEAN PATENT APPLICATION

(11) **EP 1 936 350 A1**
(43) Date of publication of application: **25.06.2008**
(21) Application number: 06126507.0
(22) Date of filing: 19.12.2006
(51) Int. Cl.: G01N 15/02, G01N 33/543, G01R 33/12

(54) **A method for quantitatively measuring agglutination parameters**

(71) Applicant: Koninklijke Philips Electronics N.V., 5621 BA Eindhoven (NL)
(72) Inventor: The designation of the inventor has not yet been filed
(74) Representative: Van Velzen, Maaike Mathilde

(57) **Abstract**

The present invention relates to a method for quantitatively measuring one or more agglutination parameters of magnetic labels (3, 15) in a target-induced agglutination assay. Magnetic labels (3, 15) and target (5) perform an agglutination process resulting in agglutinated particles (100). Thereafter a separation process in dependency on the size of the agglutinated particles takes place. For measurement of a magnetic property of the magnetic labels (15) an AC magnetic field (H_{AC}) is generated in the proximity of a magnetic sensor element (11), and the assay is positioned in the proximity of a surface of the magnetic sensor element. The measurement of a magnetic property of the magnetic labels (15) provides an indication of the agglutination parameters in a more quantitative manner than hitherto possible. The measurement of the magnetic property can e.g. be measured spatially (Fig. 10) or temporally (Fig. 11).

## Description

### FIELD OF THE INVENTION

The present invention relates to a method for quantitatively measuring one or more agglutination parameters of magnetic labels in a target-induced agglutination assay. The invention also relates to a corresponding kit, and a corresponding device.

### BACKGROUND OF THE INVENTION

Recent efforts have been given to measurement techniques for determining the presence, and possibly the level of concentration, of targets in a larger mixture or solution in which the particles reside. It is often desirable to measure relatively low concentrations of certain organic compounds. In medicine, for example, it is very useful to determine the concentration of a given kind of molecule, usually in solution, which either exists naturally in physiological fluids e.g. blood or urine or which are introduced into the living system e.g. drugs.

One broad approach used to detect the presence of a particular compound of interest is the immunoassay technique, in which detection of a given molecular species, referred to generally as the ligand, is accomplished through the use of a second molecular species, often called the antiligand or the receptor, which specifically binds to the ligand of interest. The presence of the ligand of interest is detected by measuring, or inferring, either directly or indirectly, the extent of binding of ligand to antiligand e.g. by optical methods. A ligand can be considered a target or an analyte.

There are a number of immunoassay formats. All involve bindings but not all involve agglutination. In the non-agglutinating case, one label is generally attached to one target. Agglutination assays are rapid and easy to detect, and they are used when easy detection and immediate results are required e.g. in the field.

Quantitative tests based on optical detection of large particles are only moderately sensitive because they rely on measurements of turbidity (transmitted light through sample) or nephelometry (scattered light through sample), both of which are influenced by background interference from particulate matter. Consequently, optical methods are not suitable for use with raw samples such as whole blood, which contains cells, and saliva, which may have food particles.

Instead, magnetic particles made from magnetite and inert matrix material have long been used in the field of biochemistry. They range in size from a few nanometers up to a few microns in diameter and may contain from 15% to 100% magnetite. They are often described as superparamagnetic particles or, in the larger size range, as magnetic beads. The usual methodology is to coat the surface of the particles with some biologically active material which will cause them to bind strongly with specific microscopic objects or particles of interest e.g. proteins, viruses, cells, or DNA fragments. The magnetic particles may be considered as "handles" by which the objects can be moved or immobilized using a magnetic gradient, usually provided by a strong permanent magnet.

Previously, such magnetic particles have been used primarily for immobilizing the bound objects, but recent work has been done on using the particles as tags for detecting the presence of the bound complexes. Historically the detection and quantification of the bound complexes have been accomplished by means of radioactive, fluorescent, or phosphorescent molecules which are bound to the complexes of interest. However, these prior tagging techniques have various well-known disadvantages.

On the other hand, since the signal from a tiny volume of magnetic particles is exceedingly small, it has been natural that researchers have tried building detectors based on Superconducting Quantum Interference Devices (SQUIDs), which are well known to be the most sensitive detectors of magnetic fields for many applications. However, SQUIDs are quite sensitive measurement devices, but suffer inter alia from the disadvantage that the devices have to be cooled around cryogenic temperatures.

Recently, improved magnetic particle sensor devices have been disclosed by the present applicant, in particular in international patent applications WO 2005/010542 and WO 2005/010543, which are both hereby incorporated by reference in their entirety. These magnetic particle sensor devices have the advantages that measurement can be performed at around room temperature while at the same time having a sufficiently high signal-to-noise ratio (SNR).

US 6,437,563 (to Simmonds et al. and Quantum Design, Inc.) recently disclosed an apparatus, which is provided for quantitatively measuring combinations of magnetic particles combined with analytes whose amount or other characteristic quality is to be determined. The magnetic particles are complexed with the analytes to be determined and are excited in a magnetic field of several hundred kHz. The magnetizations of the magnetic particles are thereby caused to oscillate at the excitation frequency in the manner of a dipole to create their own fields. These fields are inductively coupled to at least one sensor such as sensing coils fabricated in a gradiometer configuration. The output signals from the sensing coils are appropriately amplified and processed to provide useful output indications for combinations or agglutination of magnetic particles. However, working in the kilo-Hertz regime of oscillation may introduce unnecessary noise contributions, and additionally the application of a moving (rotating) sample holder complicates the design as the rotating sample holder has to be relatively precise when the rotation is applied for decoupling of measurement and excitation. Additionally, the rotating sample holder does not easily facilitate sample manipulation immediately prior to, during, or immediately after magnetic measurement. Moreover, the coil technology applied by Simmonds et al. is not very sensitive, and thus relatively large amount of magnetic material is required for detection, which therefore increases the need for higher sample volumes. Finally, Simmonds et al. only measures the total amount of magnetic material both in the form of clusters and single particles, and effectively they do not distinguish between the two forms making it impossible to measure agglutination parameters.

Hence, an improved method for quantitatively measuring of agglutination parameters would be advantageous, and in particular a more efficient and/or reliable method would be advantageous.

### SUMMARY OF THE INVENTION

Accordingly, the invention preferably seeks to mitigate, alleviate or eliminate one or more of the above mentioned disadvantages singly or in any combination. In particular, it may be seen as an object of the present invention to provide an improved method for quantitatively measuring of agglutination parameters that solves the above mentioned problems of the prior art with sample manipulation.

This object and several other objects are obtained in a first aspect of the invention by providing a method for quantitatively measuring one or more agglutination parameters of magnetic labels in a target-induced agglutination assay, the method comprising:
- providing magnetic labels in the assay, said magnetic labels being capable of binding to the target,
- performing an agglutination process resulting in agglutinated particles,
- performing a separation process in dependency on the size of the agglutinated particles,
- generating a magnetic field, preferably AC magnetic field, in the vicinity of a magnetic sensor element, the assay being positioned in the proximity of a surface of the magnetic sensor element, and
- measuring with the magnetic sensor element a magnetic property of the one or more magnetic labels, the magnetic property being related to the AC magnetic field, wherein the measured magnetic property is indicative of the one or more agglutination parameters.

The invention is particularly, but not exclusively, advantageous for obtaining a method for studying agglutination of magnetic labels or magnetic particles in a more quantitative manner than hitherto disclosed. In particular, this is obtained due to the low-noise magnetic measurement technique applied, and/or the improved separation of the agglutinated particles prior to and/or during measurement of the magnetic property of magnetic labels, which provides an improved sensitivity of the desired agglutination parameters. With respect to the sensitivity in terms of the number of measurable particles, experiments performed have shown that the present invention is at least an order of magnitude more sensitive than the device of Simmonds et al.

As a further advantage, the method facilitates measurement of the agglutinated particles not necessarily bound to the magnetic sensor surface. This may be particularly beneficial as complicated surface structuring (such as surface patterning and surface modification) can be superfluous, and hence this can result in significantly simplified manufacturing of magnetic sensing devices.

Agglutination parameters may include, but need not be limited to, the size of the agglutinates formed, the total amount of magnetic material in the form of agglutinates comprising more than 2 individual magnetic particles, the size distribution of the agglutinates, the magnetic particle number distribution of the individual particles forming the agglutinates, the ratio of free individual magnetic particles to agglutinated individual particles, etc.

The magnetic sensor may be any suitable sensor based on the detection of the magnetic properties of the particle e.g. a coil, a wire, magneto-resistive sensor, giant magneto-resistive sensor, magneto-strictive sensor, Hall sensor, planar Hall sensor, flux gate sensor, SQUID, magnetic resonance sensor, etc.

The magnetic labels can be detected directly by the magnetic sensing method, or the labels can be further processed prior to detection. An example of further processing is that materials are added or that the (bio) chemical or physical properties of the label are modified to facilitate detection as will be explained in more detail below.

The detection method of the present invention may used with several biochemical assay types, e.g. binding/unbinding assay, sandwich assay, competition assay, displacement assay, enzymatic assay, etc.

The method of this invention may be suited for sensor multiplexing (i.e. the parallel use of different sensors and sensor surfaces), label multiplexing (i.e. the parallel use of different types of labels) and chamber multiplexing (i.e. the parallel use of different reaction chambers). The methods described in the present invention may be used as rapid, robust, and easy to use point-of-care biosensors for small sample volumes.

The reaction chamber containing the sample may be a disposable item to be used with a compact reader, containing the one or more magnetic field generating means and one or more detection means. Also, the methods of the present invention may be used in automated high-throughput testing for centralized laboratories. In this case, the reaction chamber is e.g. a well plate or cuvette, fitting into an automated instrument.

Beneficially, the measuring with the magnetic sensor element of the magnetic property of the one or more the magnetic labels may be performed in a region above the said surface of the magnetic sensor element. Thus, the invention may be different from on-surface measurement, but can also be applied for on-surface measurement. More specifically, the measuring may be performed within a region of 10 micrometer, preferably 5 micrometer, more preferably 1 micrometer above the said surface of the magnetic sensor element. Even ranges of 200 micrometer, preferably 100 micrometer, more preferably 50 micrometer above the surface of the sensor may be applied.

In one embodiment, the direction of the generated AC magnetic field may be mainly perpendicular to the plane of the magnetic sensor element in the direct neighbourhood of the magnetic sensor element. Additionally, the magnetic sensor element may comprise magnetoresistive measuring means, preferably giant-magnetoresistive measuring means.

Alternatively, the magnetic sensor element may comprises Hall measuring means as Hall measuring means are generally easy to manufacture as compared to magnetoresistive measuring means.

Typically, the separation process may be performed within a substantially confined volume of the assay. Thus, the separation in dependency of size need not be performed in another sample container or in a different process location, but can take place where the measurement is performed.

Beneficially, the separation process may performed by magnetic forces acting on at least a portion of the magnetic labels in the assay, the magnetic forces originating from an inhomogeneous magnetic separation field (H_{SEP}). The magnetic forces applied for separation can be different from the AC magnetic field, as this facilitates a more specific separation process.

In a particular embodiment, an agglutination enhancement magnetic field (H_{ENH}) may additionally be applied for enhancing the agglutination process, the magnetic field (H_{ENH}) may be applied prior and/or simultaneously to the magnetic separation field (H_{SEP}).

The magnetic property may be measured over time for obtaining a temporal measurement. Interval of times may range from second up to an hour, but typical 1 to 10 minutes or from 1 to 5 minutes. When measuring the magnetic property over time for it may be possible to obtain an indication of a size distribution of the agglutinated particles.

Beneficially, a plurality of magnetic sensor elements may be arranged with each element having a surface in the proximity of the assay, to provide quantitative agglutination parameters resulting from the separation process according to the present invention. In particular, the plurality of sensors may be arranged in relation to the assay for measuring different size fractions of agglutinated particles resulting from the separating process.

In a second aspect, the present invention relates to a kit for quantitatively measuring one or more agglutination parameters of magnetic labels in a target-induced agglutination assay according to the method of the first aspect, the kit comprising:
- an assay comprising the target, and
- magnetic labels being capable of binding to the target.

In a third aspect, the present invention relates to a device for quantitatively measuring one or more agglutination parameters of magnetic labels in a target-induced agglutination assay, the device comprising:
- associated magnetic labels in the assay, said magnetic labels being capable of binding to the target,
- processing means for performing an agglutination process resulting in agglutinated particles,
- separation means for performing a separation process in dependency on the size of the agglutinated particles,
- a magnetic sensor element, and
- magnetic generating means for generating an AC magnetic field in the vicinity of the magnetic sensor element, the assay being positioned in the proximity of a surface of the magnetic sensor element,
wherein the magnetic sensor element is arranged for measuring a magnetic property of the one or more magnetic labels, the magnetic property being related to the AC magnetic field, wherein the measured magnetic property is indicative of the one or more agglutination parameters.

In connection with the second and third aspect of the invention, it should be stated that the reagents for performing the agglutination assay including the magnetic labels may exist in a dry form on the device. The dry reagents can then be dissolved and dispersed by the addition of a fluid sample. This is advantageous for ease-of-use and storage of the device

The first, second and third aspect of the present invention may each be combined with any of the other aspects. These and other aspects of the invention will be apparent from and elucidated with reference to the embodiments described hereinafter.

### BRIEF DESCRIPTION OF THE FIGURES

The present invention will now be explained, by way of example only, with reference to the accompanying Figures, where
Figures 1, 2, and 3 are schematic reactions of an agglutination array,
Figure 4 is a cross-sectional view of a sensor device according to an embodiment of the magnetic sensor without magnetic particles,
Figure 5 is a cross-sectional view of a sensor device according to an embodiment of the magnetic sensor with magnetic particles,
Figure 6, 7, and 8 are cross-sectional views of three embodiments of the magnetic sensor with improved resolution in the direction perpendicular to the sensor plane,
Figure 9 is a schematic illustration of the separation process according to the present invention,
Figure 10 is a first embodiment of the separation process according to the present invention,
Figure 11 is a second embodiment of the separation process according to the present invention,
Figure 12 is a measured GMR signal in a kinetic study of agglutination,
Figure 13 is a schematic illustration of the various magnetic fields applied in an embodiment of the invention in dependency of time, and
Figure 14 is a flow-chart of a method according to the invention.

### DETAILED DESCRIPTION OF AN EMBODIMENT

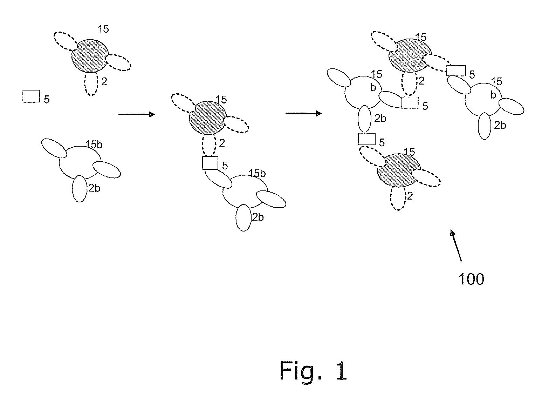

Figure 1 depicts an agglutination assay in which magnetic particle or label 15 becomes attached to magnetic particle 15b through a sandwich structure consisting of target 5 bound by binding moieties 2, and 2b attached to particle 15 and 15b, respectively. This format can be used when the target has at least two binding sites, such as protein or peptide antigens with multiple paratopes, nucleic acids (DNA, RNA), and antibodies with multiple epitopes.

Magnetic particle 15 becomes attached to magnetic particle 15b through a sandwich structure consisting of target 5 bound by moieties 2, and 2b attached to particle 15 and 15b, respectively, resulting in formation of agglutinated particles 100.

The two binding sites can be distinct, in which case binding moieties 2 and 2b are different. Alternatively, the two binding sites can be identical, in which case 2 and 2b are the same. Binding moieties 2 and 2b can be directly attached to the magnetic particles or through some intermediate binding groups (protein G, streptavidin, biotin, protein A, IgG antibodies etc.). One or both of the binding moieties 2 and 2b can be present already attached to the particle or independent of the particles in which case both the binding moieties and particles must have on them intermediate binding moieties that allow the particles to attach to the binding moieties 2 and 2b. Thus, the binding of the magnetic particle 15 and the target 5 can be indirect in the context of the present invention.

For targets 5 which are small and only have one binding site (such as small molecules, hapten, drugs, hormones, and metabolites) an inhibition or competitive format can alternatively be employed.

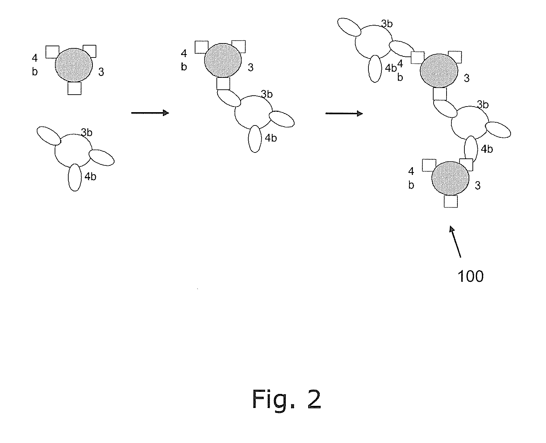

Figure 2 depicts such an assay in which magnetic particle 3 aggregates with particle 3b through moiety 4, attached to 3, which can be bound by binding-moiety 4b attached to particle 3b. Moiety 4 can be a target homologue and when the target is added, it binds to moiety 4b and inhibits the aggregation of the particles. It is preferable that the target is added before aggregation has begun, because the aggregation may be irreversible or the aggregates difficult to disperse. This can be achieved by spatially separating moiety 4 and 4b and letting the target first react with 4b. Similar to the sandwich format, binding moiety 4b can be independent of the particle and be attached during the reaction by intermediate binding moieties.

The binding moieties are specifically designed to recognize the target and can be molecules such as antibodies, nucleic acids, aptamers, peptides, proteins, and lectins. The components can bind in any order, although it is preferable to have the binding moieties already attached to the particles before exposure to the target for actuation purposes. Particles can be up to 2 micrometer, with a preference for particles less than 1 micrometer due to their larger surface-to-volume ratio which will result in a higher dynamic range. The agglutination arises from the binding of multiple particles to one another, and results in agglutinated particles 100.

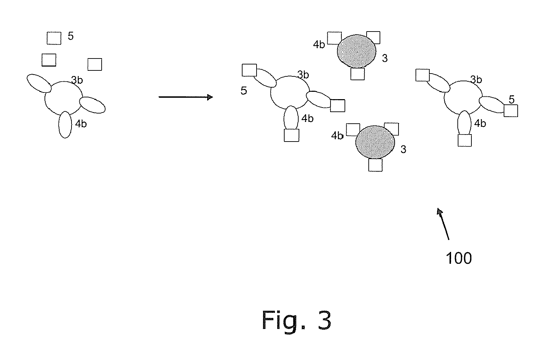

Figure 3 depicts an assay, where moiety 4 can be a target homologue, and when the target 5 is added, it binds to moiety 4b and inhibits the aggregation of the particles. Thus, it is to be understood that within the context of the present invention, an agglutination process includes a process where inhibition of agglutination is studied.

The invention is based inter alia on the insight that in the low frequency regime, at frequencies e.g. below 100 Hz, the 1/f noise of the magnetic sensor element dominates. 1/f noise is caused by point-to-point fluctuations of the current and is proportional to the inverse of the frequency. In magnetoresistive sensors, 1/f noise originates from magnetic fluctuations in the free layer. When the frequency of the generated ac magnetic field is 100 Hz or above, the dominating 1/f noise is significantly reduced compared to the prior art, resulting in an improved signal to noise ratio (SNR).

It is advantageous when the frequency of the AC magnetic field is further increased to a value where the thermal white (Nyquist) noise level becomes dominant over the 1/f noise level. As mentioned in WO 2005/010542, above a certain corner frequency f_{c}≈ 50 kHz the thermal white noise of GMR sensors becomes dominant. The white-noise level limits the theoretically achievable detection limit.

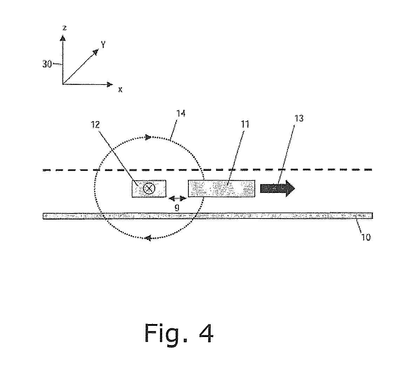

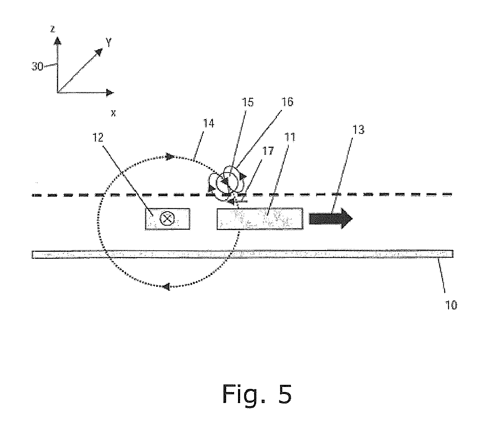

Figure 4 and 5 are cross-sectional views of a sensor device according to an embodiment of the magnetic sensor without and with, respectively, magnetic particles 15. For illustrative purposes the invention will be explained below with respect to a biosensor.

The biosensor detects magnetic particles in a sample such as a fluid, a liquid, a gas, a visco-elastic medium, a gel or a tissue sample. The magnetic particles can have small dimensions. With nanoparticles are meant particles having at least one dimension ranging between 0.1 nm and 1000 nm, preferably between 3 nm and 500 nm, more preferred between 10 nm and 300 nm. The magnetic particles can acquire a magnetic moment due to an applied magnetic field (e.g. they can be paramagnetic) or they can have a permanent magnetic moment. The magnetic particles can be a composite, e.g. consist of one or more small magnetic particles inside or attached to a non-magnetic material. As long as the particles generate a non-zero response to the frequency of an AC magnetic field, i.e. when they generate a magnetic susceptibility or permeability, they can be used.

The device may comprise a substrate 10 and a circuit e.g. an integrated circuit. A measurement surface of the device is represented by the dotted line in Fig. 4 and Fig. 5. In embodiments of the present invention, the term "substrate" may include any underlying material or materials that may be used, or upon which a device, a circuit or an epitaxial layer may be formed. In other alternative embodiments, this "substrate" may include a semiconductor substrate such as e.g. a doped silicon, a gallium arsenide (GaAs), a gallium arsenide phosphide (GaAsP), an indium phosphide (InP), a germanium (Ge), or a silicon germanium (SiGe) substrate. The "substrate" may include for example, an insulating layer such as a SiO₂ or an Si₃N₄ layer in addition to a semiconductor substrate portion. Thus, the term substrate also includes glass, plastic, ceramic, silicon-on-glass, silicon-on sapphire substrates. The term "substrate" is thus used to define generally the elements for layers that underlie a layer or portions of interest. Also, the "substrate" may be any other base on which a layer is formed, for example a glass or metal layer.

The circuit may comprise a magnetoresistive sensor 11 as a sensor element and a magnetic field generator in the form of a conductor 12. The magnetoresistive sensor 11 may for example be a GMR, a AMR or a TMR type sensor. The magnetoresistive sensor 11 may for example have an elongated, e.g. a long and narrow stripe geometry but is not limited to this geometry. Sensor 11 and conductor 12 may be positioned adjacent to each other (Fig. 4) within a close distance g. The distance g between sensor 11 and conductor 12 may for example be between 1 nm and 1 mm; e.g. 3 qm.

In Fig. 4 and 5, a co-ordinate system is introduced to indicate that if the sensor device is positioned in the xy plane, the sensor 11 mainly detects the x-component of a magnetic field, i.e. the x-direction is the sensitive direction of the sensor 11. The arrow 13 in Fig. 4 and Fig. 5 indicates the sensitive x-direction of the magnetoresistive sensor 11 according to the present invention. Because the sensor 11 is hardly sensitive in a direction perpendicular to the plane of the sensor device, in the drawing the vertical direction or z-direction, a magnetic field 14, caused by a current flowing through the conductor 12, is not detected by the sensor 11 in absence of magnetic nano-particles 15. By applying a current to the conductor 12 in the absence of magnetic nano-particles 15, the sensor 11 signal may be calibrated. This calibration is preferably performed prior to any measurement.

When a magnetic material (this can e.g. be a magnetic ion, molecule, nano-particle 15, a solid material or a fluid with magnetic components) is in the neighbourhood of the conductor 12, it develops a magnetic moment m indicated by the field lines 16 in Fig. 5. The magnetic moment m then generates dipolar stray fields, which have in-plane magnetic field components 17 at the location of the sensor 11. Thus, the nano-particle 15 deflects the magnetic field 14 into the sensitive x-direction of the sensor 11 indicated by arrow 13 (Fig. 5). The x-component of the magnetic field Hₓ which is in the sensitive x-direction of the sensor 11, is sensed by the sensor 11 and depends on the number Nₙₚ of magnetic nano-particles 15 and the conductor current I_{c}.

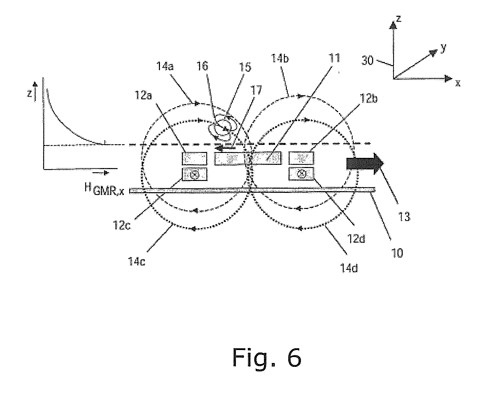

Figure 6, 7, and 8 are cross-sectional views of three embodiments of the magnetic sensor with improved resolution in the direction perpendicular to the sensor plane. In this embodiment of the present invention, an improved sensor device with respect to the previous embodiment is described. In order to distinguish between surface- and bulk concentrations of magnetic particles 15, resolution in a direction perpendicular to the plane of the sensor element 11, which corresponds to the z-direction with the co-ordinate system introduced in Fig. 4, is required. As shown in Fig. 6 conductors 12c and 12d generate a magnetic field 14c and 14d respectively in comparison with the magnetic field 14a and 14b of conductors 12a and 12b. By combining the sensor signals originating from the four conductors 12a, 12b, 12c, 12d, information may be obtained about the concentration of the magnetic particles 15 in x and z direction.

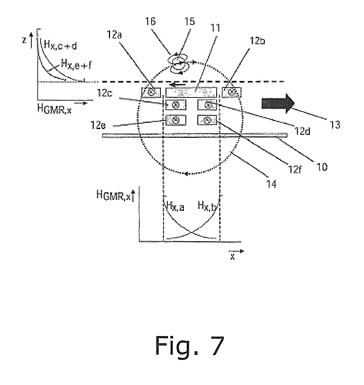

The z-resolution can be further enhanced by applying more conductors in the direction perpendicular to the plane of the sensor element 11, which as represented is the vertical or z direction. This is shown in the embodiment of Fig. 7. Conductors 12a and 12b are positioned at both sides next to the magnetic sensor 11, at the same level in a direction perpendicular to the plane of the sensor element 11. Conductors 12c, 12d, 12e and 12f are positioned between the substrate 10 and the sensor 11, the conductors 12c and 12d are at a different z-position with respect to conductors 12e and 12f. Again, combination of the sensor signals resulting from the different conductors 12a to 12f may give information about the bulk, near-surface and surface concentration of the magnetic particles 15.

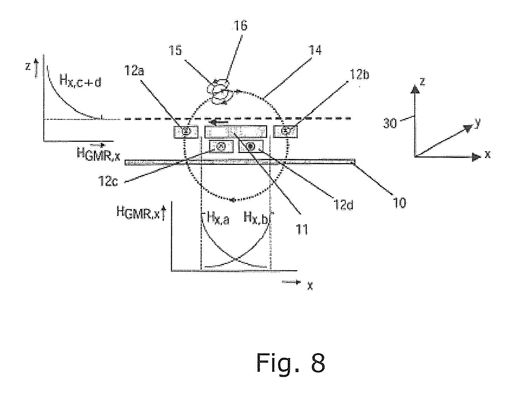

In still another embodiment, the currents in conductors 12c and 12 d, which are positioned at a level in between the substrate 10 and the magnetic sensor 11, have opposite directions, as illustrated in Fig. 8. In that way, conductors 12c and 12d may generate a strong field gradient in the x direction. This embodiment may be advantageous for enhancing spatial resolution.

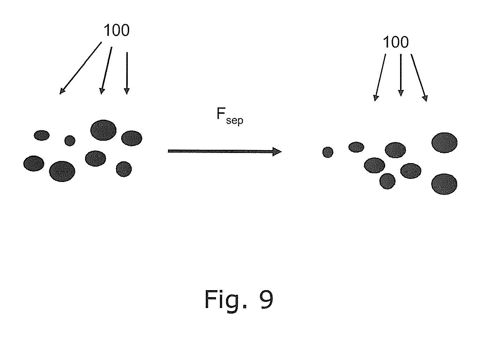

Figure 9 is a schematic illustration of the separation process according to the present invention for quantitatively measuring one or more agglutination parameters of magnetic labels 15 in a target-induced agglutination assay. After providing magnetic labels in the assay, said magnetic labels being capable of binding to the target and performing an agglutination process resulting in agglutinated particles 100, there a separation process in dependency on the size of the agglutinated particles is performed as illustrated in Figure 9. In the separation process, a separation force F_{SEP} is applied in one or more spatial directions, the separation force being dependent on the size of the agglutinated particles 100.

Preferably, the separation force F_{SEP} is an inhomogeneous magnetic field applied on the agglutinated particles 100, but other kinds of separation are also possible: rotation causing a centrifugal force to separate the forces by mass, application of a hydrodynamic pressure, application of an electrostatic separation (requires a charge on the agglutinated particles 100), etc. In addition to the use of various forces applied for separation, size exclusion filters can also be used. It may be assumed for most agglutination assays that the number of magnetic particles 15 incorporated into the agglutinated particles 100 is directly dependent, e.g. proportionally, on the size of the agglutinated particles 100, though it may be not always be the case.

With an inhomogeneous magnetic field applied for separation, the magnetic field may be generated by the same means generating the AC magnetic field, or alternatively by dedicated magnetic generating means may be arranged close the magnetic sensor element 11 for that purpose.

The generated AC magnetic field in the vicinity of the magnetic sensor element 11 enables measurements of a magnetic property of the one or more magnetic particles 15, where the magnetic property is related to the AC magnetic field. The magnetic property and the corresponding detected signal can be correlated to the number of magnetic particles with a certain detection range of the sensor element 11, and due to the separation process performed, the number of particles thereby detected can be transformed into a size distribution.

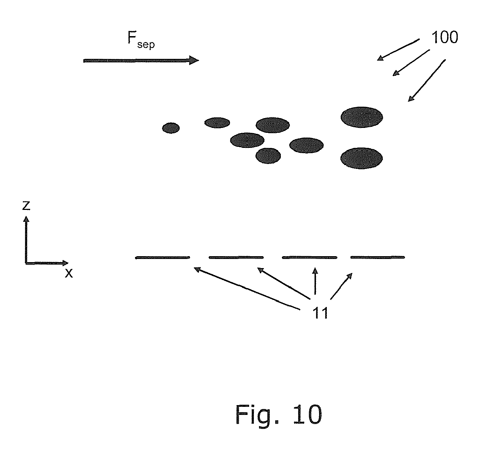

Figure 10 is a first embodiment of the separation process according to the present invention, where a plurality of sensor elements 11 are arranged relative to the separated agglutinated particles 100 to facilitate a measurement of the size distribution of the agglutinated particles as illustrated in Figure 10. The separation results in a spatial separation with respect to the different sensor elements 11 in one spatial direction, but similarly the separation can be performed also in two or three spatial dimensions.

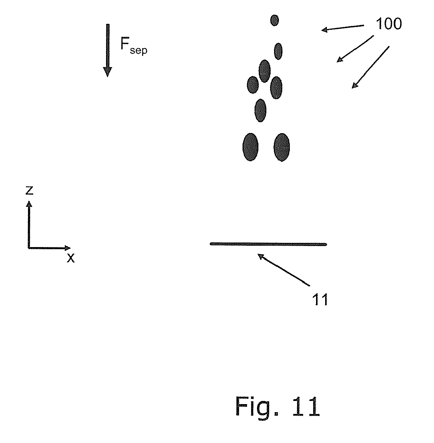

Figure 11 is a second embodiment of the separation process according to the present invention, where a single sensor 11 is arranged relatively to the separation force F_{SEP} applied so that a measurement over time will reveal, or at least indicate, the size distribution of the agglutinated particles 100. The separation can be characterized as a temporal separation. Time dependent measurement by the sensor 11 can also be applied for measurement of agglutination kinetics.

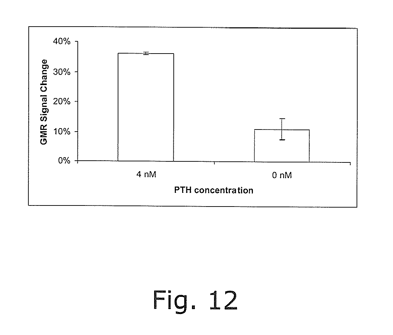

Figure 12 is an example of a kinetic study with a parathyroid hormone assay in which 4 nM of the analyte is first added to particles containing antibodies to PTH. The measurement is performed in a setup similar to Figure 11 for 60 minutes. The particles aggregate in the presence of the target (200 nm) and sediment faster under weak magnetic actuation than the sample containing no analyte, the result being a significantly larger GMR signal. The separation force F_{SEP} is made by an alternating current (AC) magnetic field.

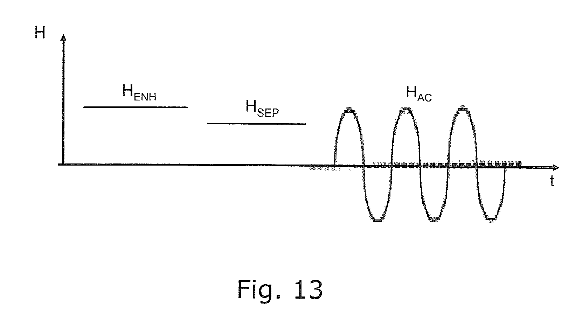

Figure 13 is a schematic illustration of the various magnetic fields applied in an embodiment of the invention in dependency of time, t. Only the numerical values of the magnetic fields are indicated as the three magnetic fields could have different direction from one another. At first a magnetic field is applied for enhancement of the agglutination process, i.e. by applying an inhomogeneous magnetic field H_{ENH} across the assay, the magnetic particles are actuated, and hence agglutination is increased by reversible non-specific interaction.

Thus, the particles containing the binding moieties can be moved through the solution with magnetic actuation so that the number of target encounters with the binding moieties is increased. This can be used in order to enhance the speed of the assay or enhance its sensitivity. Although this can be done during agglutination, actuation is preferably performed before agglutination to have as much of the binding moieties exposed as possible. This can be achieved by exposing the target 5 first to the particle 15-binding moiety 2 complex, actuating and then exposing the target to the particle 15b-binding moiety 2b complex or vice versa, cf. Figure 1. A similar scheme is possible for the inhibition format.

In an alternative embodiment, enhancing the formation of agglutinates is performed by enhancing the magnetic particle 15 encounter with the other magnetic particles 15b after the target 5 has been attached to the binding moiety 2 and 2b, cf. Figure 1. The magnetic forces involved do not have to be the same for this and the previous embodiment of enhancement but the magnetic field can be same for some embodiments. Although both types of enhancement can be formed simultaneously, it is preferable to perform the first form of enhancement, then add particle 15b and then perform the second form of enhancement

Magnetization of the particles is necessary for actuation. This may result in reversible aggregation of the particles under the external field. It is highly preferable to perform the assay under buffer conditions that prevent the clusters from permanently binding to one another non-specifically. The buffer can contain at least 1 % proteins that prevent non-specific binding (albumins, globulin, gelatin, casein, etc.) and/or at least 0.05 % detergents (Triton X-100, Tween 20 or 80 etc) and/or polymers (PVB, PEG etc.).

Separation may then be performed by applying the magnetic field H_{SEP} on the assay as described above. Both the separation magnetic field H_{SEP} and the enhancement magnetic field H_{ENH} are subject to the conditions that the fields should not be too strong or have too strong spatial gradients to prevent irreversible non-specific agglutination that will distort the measurement. With 300 nm magnite particles, the inventors have found that upper limits of the field strength of 1 × 10^4 A/m, 1 x 10^5 A/m, or 1 x 10^6 A/m are suitable, whereas upper limits for the gradient may be 1 x 10^7 A/m^2, 1 x 10^8 A/m^2 or 1 x 10^9 A/m^2. Typically, the magnetic field for separation H_{SEP} will be an order of magnitude below these upper limits.

In Figure 13, the magnetic field H_{SEP} and magnetic field H_{ENH} are indicated as being constant over time, but they could also be time-dependent, and alternating in direction like the magnetic field H_{AC} for measurement of the one or more agglutination parameters.

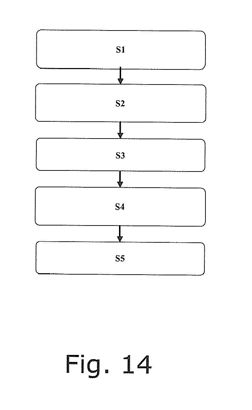

Figure 14 is a flow-chart of a method according to the invention for quantitatively measuring one or more agglutination parameters of magnetic labels 3 or 15 in a target-induced agglutination assay, the method comprising:
- **S1** providing magnetic labels 3 or 15 in the assay, said magnetic labels being capable of binding to the target 5,
- **S2** performing an agglutination process resulting in agglutinated particles 100,
- **S3** performing a separation process in dependency on the size of the agglutinated particles by applying a separation force F_{SEP},
- **S4** generating an AC magnetic field H_{AC} in the vicinity of a magnetic sensor element 11, the assay being positioned in the proximity of a surface of the magnetic sensor element, and
- **S5** measuring with the magnetic sensor element a magnetic property of the one or more magnetic labels or particles 3 or 15, the magnetic property being related to the AC magnetic field H_{AC},
wherein the measured magnetic property is indicative of the one or more agglutination parameters.

The invention can be implemented in any suitable form including hardware, software, firmware or any combination of these. The invention or some features of the invention can be implemented as computer software running on one or more data processors and/or digital signal processors. The elements and components of an embodiment of the invention may be physically, functionally and logically implemented in any suitable way. Indeed, the functionality may be implemented in a single unit, in a plurality of units or as part of other functional units. As such, the invention may be implemented in a single unit, or may be physically and functionally distributed between different units and processors.

Although the present invention has been described in connection with the specified embodiments, it is not intended to be limited to the specific form set forth herein. Rather, the scope of the present invention is limited only by the accompanying claims. In the claims, the term "comprising" does not exclude the presence of other elements or steps. Additionally, although individual features may be included in different claims, these may possibly be advantageously combined, and the inclusion in different claims does not imply that a combination of features is not feasible and/or advantageous. In addition, singular references do not exclude a plurality. Thus, references to "a", "an", "first", "second" etc. do not preclude a plurality. Furthermore, reference signs in the claims shall not be construed as limiting the scope.

## Claims

1. A method for quantitatively measuring one or more agglutination parameters of magnetic labels (3, 15) in a target-induced agglutination assay, the method comprising:
- providing magnetic labels (3, 15) in the assay, said magnetic labels being capable of binding to the target (5),
- performing an agglutination process resulting in agglutinated particles (100),
- performing a separation process in dependency on the size of the agglutinated particles,
- generating a magnetic field in the vicinity of a magnetic sensor element (11), the assay being positioned in the proximity of a surface of the magnetic sensor element, and
- measuring with the magnetic sensor element a magnetic property of the one or more magnetic labels (3, 15), the magnetic property being related to the AC magnetic field (H_{AC}),
wherein the measured magnetic property is indicative of the one or more agglutination parameters.

2. A method according to claim 1, wherein the magnetic field is an AC field (H_{AC}).

3. A method according to claim 1, wherein the measuring with the magnetic sensor element (11) of the magnetic property of the one or more magnetic labels (3, 15) is performed in a region above the said surface of the magnetic sensor element.

4. A method according to claim 3, wherein the measuring is performed within a region of 10 micrometer, preferably 5 micrometer, more preferably 1 micrometer above the said surface of the magnetic sensor element (11).

5. A method according to claim 1, wherein the direction of the generated magnetic field is mainly perpendicular to the plane of the magnetic sensor element (11) n the direct neighbourhood of the magnetic sensor element.

6. A method according to claim 1 or 5, wherein the magnetic sensor element (11) comprises magnetoresistive measuring means.

7. A method according to claim 1 or 5, wherein the magnetic sensor element (11) comprises Hall measuring means.

8. A method according to claim 1, wherein the separation process is performed within a substantially confined volume of the assay.

9. A method according to claim 1, wherein the separation process is performed by magnetic forces acting on at least a portion of the magnetic labels in the assay, said magnetic forces originating from an inhomogeneous magnetic separation field (H_{SEP}).

10. A method according to claim 9, wherein the magnetic forces (H_{SEP}) applied for separation are different from the magnetic field.

11. A method according to claim 9, wherein an agglutination enhancement magnetic field (H_{ENH}) is applied for enhancing the agglutination process, said magnetic field (H_{ENH}) being applied prior to and/or simultaneously with the magnetic separation field (H_{SEP}).

12. A method according to claim 1, wherein the magnetic property is measured over time.

13. A method according to claim 12, wherein the magnetic property is measured over time for obtaining an indication of a size distribution of the agglutinated particles (100).

14. A method according to claim 1, wherein a plurality of magnetic sensor elements (11) are arranged with each element having a surface in the proximity of the assay.

15. A method according to claim 14, wherein the plurality of sensors are arranged in relation to the assay for measuring different size fractions of agglutinated particles (100) resulting from the separating process.

16. A method according to claim 1, wherein the assay is a biochemical assay.

17. A kit for quantitatively measuring one or more agglutination parameters of magnetic labels (15) in a target-induced agglutination assay according to the method of claim 1, the kit comprising:
- an assay comprising the target (5), and
- magnetic labels (3, 15) being capable of binding to the target (5).

18. A device for quantitatively measuring one or more agglutination parameters of magnetic labels (3, 15) in a target-induced agglutination assay, the device comprising:
- associated magnetic labels (3, 15) in the assay, said magnetic labels being capable of binding to the target (5),
- processing means for performing an agglutination process resulting in agglutinated particles (100),
- separation means (12) for performing a separation process in dependency on the size of the agglutinated particles,
- a magnetic sensor element (11), and
- magnetic generating means (12) for generating a magnetic field in the vicinity of the magnetic sensor element, the assay being positioned in the proximity of a surface of the magnetic sensor element,
wherein the magnetic sensor element (11) is arranged for measuring a magnetic property of the one or more magnetic labels (3, 15), the magnetic property being related to the AC magnetic field, wherein the measured magnetic property is indicative of the one or more agglutination parameters (100).
